(19) Europäisches Patentamt
European Patent Office
Office européen des brevets

(11) Veröffentlichungsnummer: **0 028 777**
**B1**

(12) # EUROPÄISCHE PATENTSCHRIFT

(45) Veröffentlichungstag der Patentschrift: **30.03.83**

(51) Int. Cl.³: **C 07 D221/14**, D 06 L 3/12// D21H3/80

(21) Anmeldenummer: **80106703.4**

(22) Anmeldetag: **31.10.80**

(54) **Kationische Verbindungen der Naphthalimid-Reihe, Verfahren zu deren Herstellung und deren Verwendung.**

(30) Priorität: **07.11.79 DE 2944867**

(43) Veröffentlichungstag der Anmeldung: **20.05.81 Patentblatt 81/20**

(45) Bekanntmachung des Hinweises auf die Patenterteilung: **30.03.83 Patentblatt 83/13**

(84) Benannte Vertragsstaaten: **AT BE CH DE FR GB IT LI NL SE**

(56) Entgegenhaltungen:
**CH A 580 134**
**DE A 1 419 350**
**DE B 1 795 091**
**FR A 1 344 883**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt/Main 80 (DE)**

(72) Erfinder: **Schönberger, Norbert, Dr.**
**Tilsiter Weg 3**
**D-6393 Wehrheim (DE)**
Erfinder: **Schinzel, Erich, Dr.**
**Ostpreussenstrasse 43**
**D-6238 Hofheim am Taunus (DE)**
Erfinder: **Martini, Thomas, Dr.**
**Am Schellberg 42**
**D-6232 Bad Soden am Taunus (DE)**
Erfinder: **Rösch, Günter**
**Hohlweg 17**
**D-6232 Bad Soden am Taunus (DE)**

EP 0 028 777 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Kationische Verbindungen der Naphthalimid-Reihe, Verfahren zu deren Herstellung und deren Verwendung

Kationische optische Aufheller der Naphthalimid-Reihe, die quartäre Stickstoffatome enthalten, sind bereits bekannt. So werden in dem Franz. Patent 1 557 945 kationische Naphthalimid-Derivate beschrieben, welche Trialkylammonium-Reste über eine Kette von zwei oder drei Methylengruppen am Imid-Stickstoff gebunden enthalten.

Gegenstand der Erfindung sind neue kationische Verbindungen der allgemeinen Formel (1)

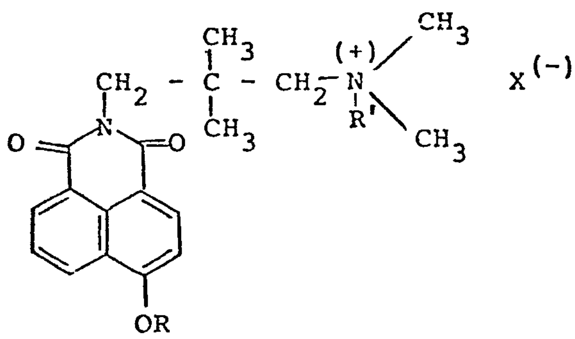

(1)

in welcher

R Alkyl, Hydroxyalkyl, Alkoxyalkyl, Phenylalkyl oder Phenoxyalkyl,

R' Alkyl, Hydroxyalkyl, Benzyl oder Allyl und

$X^{(-)}$ ein farbloses Anion bedeutet.

Die Rest R und R' enthalten 1-4 C-Atome in den Alkyl- bzw. Alkoxygruppen wie beispielsweise Ethyl, Propyl, Butyl, Hydroxyethyl, Methoxyethyl, Ethoxyethyl, Propoxyethyl, Phenoxyethyl oder Benzyl und insbesondere aufgrund der günstigen Herstellungsmöglichkeiten Methyl. $X^{(-)}$ ist beispielsweise ein Halogenid, Alkylsulfonat-, Alkylsulfat-, Alkylphenylsulfonat- oder Phenylsulfonat-Ion.

Die Herstellung der Verbindungen der Formel (1) erfolgt in bekannter Weise durch Alkylierung einer Verbindung der Formel (2)

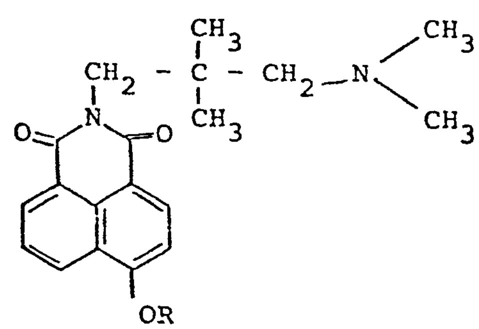

(2)

mit einer Verbindung der Formel

$$X—R'.$$

Diese Alkylierung erfolgt vorzugsweise in einem geeigneten inerten organischen Lösungsmittel wie Toluol, Xylol, Chlorbenzol, Dimethylformamid oder Tetralin bei Temperaturen von Raumtemperatur bis 100°, vorzugsweise Raumtemperatur bis 60 °C.

Die als Ausgangsmaterialien benötigten Naphthalimide (2) werden erhalten durch Umsetzung der 4-Chlor- oder Bromnaphthalsäureanhydride mit N,N,2,2-Tetramethylpropan-1,3-diamin (Dimethylamino-neopentylamin) zu den entsprechenden N-(2,2-Dimethyl-3-dimethylaminopropyl)-4-halogennaphthalimiden und anschließenden Austausch der Halogenatome durch Reaktion mit den Alkoholen HOR bzw. deren Alkalisalzen.

Die neuen Verbindungen dienen zum Aufhellen von organischen Materialien, wie etwa Baumwolle, vor allem von synthetischen Fasern, z. B. von Polyamiden wie Polymeren auf Basis von Hexamethylendiaminadipat oder Caprolactam, Celluloseestern wie Cellulose-2 1/2-Acetat und Cellulosetriacetat, und insbesondere aus Polyacrylnitril.

Man kann das organische Material beispielsweise dadurch aufhellen, daß man diesem geringe Menger erfindungsgemäßer optischer Aufheller, zweckmäßig 0,001 bis 1 %, bezogen auf das aufzuhellende Material, gegebenenfalls zusammen mit anderen Substanzen wie Weichmachern, Stabilisatoren

oder Pigmenten, einverleibt. Man kann die Aufheller beispielsweise gelöst in Weichmachern, wie Dioctylphthalat, oder zusammen mit Stabilisatoren, wie Dibutylzinndilaurat oder Natrium-pentaoctyltripolyphosphat, oder zusammen mit Pigmenten, beispielsweise Titandioxid, in die Kunststoffe einarbeiten. Je nach der Art des aufzuhellenden Materials kann der Aufheller auch in den Monomeren vor der Polymerisation, in der Polymerenmasse oder zusammen mit den Polymeren in einem Lösungsmittel gelöst werden.

Das so vorbehandelte Material wird hierauf nach an sich bekannten Verfahren, wie Verspinnen und Strecken, in die gewünschte endgültige Form gebracht. Man kann die Aufheller auch in Appreturen einarbeiten, beispielsweise in Appreturen für Textilfasern wie Polyvinylalkohol, oder in Harze bzw. Harzvorkondensate wie z. B. Methylolverbindungen von Ethylenharnstoff, die zur Textilbehandlung dienen.

Die erfindungsgemäßer Verbindungen eignen sich auch zum Aufhellen von Papier im Oberflächenstrich. Vorzugsweise wird jedoch farbloses, hochmolekulares organisches Material in Form von fasern aufgehellt. Zum Aufhellen dieser Fasermaterialien verwendet man vorteilhaft eine wäßrige Lösung der erfindungsgemäßen Verbindungen der Formel (1). Diese Lösungen weise hierbei vorzugsweise einen Gehalt von 0,005 bis 0,5 % an erfindungsgemäßen Naphthalimiden, bezogen auf das Fasermaterial, auf. Daneben können diese Lösungen Hilfsstoffe enthalten, wie Dispergatoren, beispielsweise Kondensationsprodukte, 10 bis 18 Kohlenstoffatome aufweisender Fettalkohole oder Alkylphenole mit 15 bis 25 Mol Ethylenoxyd, oder Kondensationsprodukte, 16 bis 18 Kohlenstoffatome aufweisender Alkylmono- oder Polyamine mit mindestens 10 Mol Ethylenoxyd, organische Säuren wie Ameisen-, Oxal- oder Essigsäure, Waschmittel, Quellmittel wie Di- oder Trichlorbenzole, Netzmittel wie Sulfobernsteinsäure-alkylester, Bleichmittel wie Natriumchlorit, Peroxide oder Hydrosulfite, Retarder, sowie gegebenenfalls Aufhellungsmittel der gleichen oder anderer Klassen, wie z. B. celluloseaffine Derivate des Stilbens.

Die Aufhellung des Fasermaterials mit der wäßrigen Aufhellerflotte erfolgt entweder im Ausziehverfahren bei Temperaturen von vorzugsweise 30 bis 150 °C oder im Foulardverfahren. Im letzteren Falle imprägniert man die Ware mit einer beispielsweise 0,2 bis 0,5 %igen Aufhellerlösung und stellt das Färbegut z. B. durch trockene oder feuchte Hitzebehandlung fertig, beispielsweise durch Dämpfen bei 2 Atmosphären oder nach erfolgter Trocknung durch kurzes trokkenes Erhitzen auf 180 bis 220 °C, wobei gegebenenfalls das Gewebe zugleich thermofixiert wird. Das derart behandelte Fasermaterial wird zum Schluß gespült und getrocknet.

Erfindungsgemäß optisch aufgehelltes, farbloses, hochmolekulares, organisches Material, insbesondere das nach dem Ausziehverfahren aufgehellte natürliche oder synthetische Fasermaterial, weist ein gefälliges, rein weißes, blauviolett bis blaustichig fluoreszierendes Aussehen auf, in hellen Farbtönen gefärbtes und erfindungsgemäß weißgetöntes derartiges Material zeichnet sich durch reinen Farbton aus.

Waschflotten, die Naphthalimide der Formel (1) enthalten, verleihen beim Waschen den damit behandelten Textilfasern, beispielsweise synthetischen Polyamid- und Celluloseesterfasern, insbesondere aber Polyacrylnitrilfasern, einen brillanten Aspekt im Tageslicht.

Hervorzuheben sind vor allem die gute Lichtechtheit, die hohe Chloritbeständigkeit, die hohe Farbstärke und insbesondere der hohe Weißgrad, sowie das insgesamt ausgezeichnete färberische Verhalten der erfindungsgemäßen Verbindungen (1).

Die Verbindungen der Formel (1), die sowohl in β-Stellung zu der quartären Ammoniumgruppe, als auch in β-Stellung zum Imid-Stickstoff ein quartäres Kohlenstoffatom enthalten, erweisen sich als außerordentlich stabil. Sie sind den eingangs erwähnten kationischen Naphthalimiden, welche Trialkylammonium-Reste über eine Kette von zwei oder drei Methylengruppen am Imid-Stickstoff gebunden enthalten, in ihrer aufhellenden Wirlung, insbesondere in der einbadigen Chloritbleiche der Polyacrylnitrilfaser, deutlich überlegen.

Beispiel 1

Zur Herstellung der Verbindung

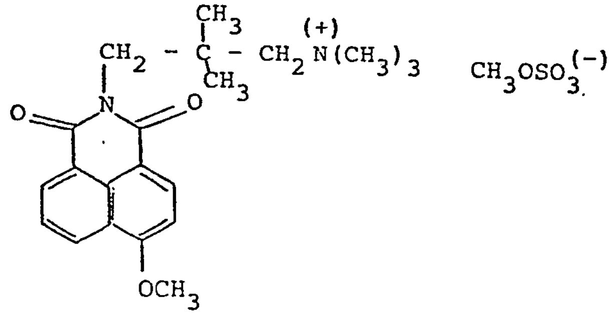

werden 17 g des 4-Methoxy-N-dimethylamino-neopentyl-naphthalimides in 100 ml Aceton gelöst und bei

Raumtemperatur mit 5,2 ml Dimethylsulfat tropfenweise versetzt. Man rührt 18 Stunden bei Raumtemperatur nach, saugt ab und wäscht mit Aceton nach. Nach dem Trocknen bei 60° im Vakuum werden 21,2 g eines gelblichen Kristallpulvers erhalten, entsprechend 91 % der Theorie. Schmelzpunkt 231-233°, S. 228°.

Das als Ausgangsmaterial benötigte 4-Methoxy-N-dimethylamino-neopentyl-naphthalimid wird in folgender Weise gewonnen : In einem Rührautoklaven werden in 750 ml Methanol 121 g 4-Chlornaphthalsäureanhydrid und 81,4 ml Dimethylamino-neopentylamin eingetragen. Man erhitzt auf 70° und hält 5 Stunden bei dieser Temperatur. Anschließend läßt man auf 50° erkalten, setzt 60 g 40 %iger Natronlauge zu und heizt auf 100° auf. Bei dieser Temperatur wird das Reaktionsgemisch 2 Stunden verrührt. nach dem Erkalten auf Raumtemperatur wird das ausgefallene Reaktionsprodukt abgesaugt, mit 500 ml Methanol und anschließend mit Wasser portionsweise gewaschen. Man trocknet bei 60° im Vakuum und erhält 142,0 g eines gelben Pulvers vom Schmelzpunkt 107-110°, entsprechend einer Ausbeute von 83,5 % der Theorie.

## Beispiel 2

In 200 ml Wasser werden 0,24 g Natriumchlorit, 0,1 g eines handelsüblichen Bleichhilfsmittels und 0, 3 ml 2n Essigsäure bei 60 °C gelöst. Vom optischen Aufheller gemäß Beispiel 1 wird eine Lösung hergestellt, indem man 0,02 g in 1 ml DMF löst. Man gießt diese Lösung in das wäßrige Bad und stellt durch Zutropfen von Essigsäure (10 %ig) den pH-Wert auf 3,5. In diese wäßrige, den Aufheller enthaltende Flotte gibt man bei 60 °C ein 5 g schweres Polyacrylnitrilgewebe. Man bleicht zuerst eine halbe Stunde bei 80 °C vor und färbt noch eine halbe Stunde bei 100 °C nach. Es wird heiß und kalt mit Wasser gespült und getrocknet. Das so behandelte Fasermaterial zeigt ein gefälliges, weißes Aussehen mit einer violetten Nuance. Eine ähnliche Wirkung erreicht man wenn man in Abwesenheit von Natriumchlorit und des Bleichhilfsmittels arbeitet und den pH-Wert der Flotte mit Essigsäure (10 %ig) auf 4 stellt.

## Beispiel 3

Zu 100 ml Wasser werden 0,12 ml 85 %ige Ameisensäure gegeben. Vom optischen Aufheller gemäß Beispiel 1 wird eine Lösung hergestellt, indem man 1 g in 100 ml Wasser löst. Von dieser Stammlösung gibt man 1,5 ml zu der oben beschriebenen Lösung. Die so erhaltene Flotte wird auf 60 °C erwärmt und in diese ein 3 g schweres Polyacrylnitrilgewebe gegeben. Man steigert die Temperatur innerhalb von 10 bis 15 Minuten auf 95 bis 98 °C und beläßt bei dieser Temperatur eine Stunde. Das Gewebe wird sodann 2 Minuten im fließenden kalten Wasser gespült und anschließend bei 60° getrocknet. Das so behandelte Gewebe zeigt ein weißes brillantes Aussehen.

In der gleichen Weise wie in Beispiel 1 beschrieben, erhält man die in der folgenden Tabelle aufgeführten Verbindungen :

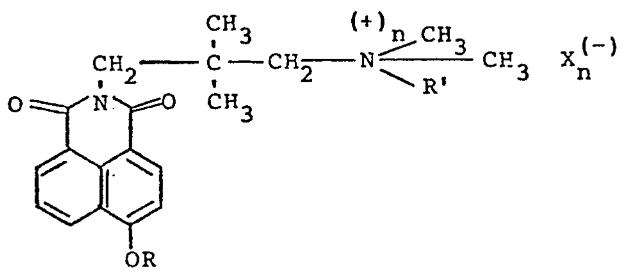

| R | R' | $X^{(-)}$ | n | Reaktionsmedium Reaktionstemperatur | Schmelzpunkt (Reinigungsmittel) |
|---|---|---|---|---|---|
| $C_2H_5$- | $HOCH_2CH_2$- | $Cl^{(-)}$ | 1 | 2-Chlorethanol 128° | 204,5° u. Zers. (aus Pyridin) |
| $CH_3CH_2CH_2$- | $CH_3$ | $CH_3OSO_3^{(-)}$ | | Toluol 60° | 184 - 192° |
| $CH_3CH_2CH_2$- | $HOCH_2CH_2$- | $Cl^{(-)}$ | | 2-Chlorethanol 128° | 113 - 116,5° (aus Ethanol) |

(Fortsetzung)

| R | R' | $X^{(-)}$ | n | Reaktionsmedium Reaktionstemperatur | Schmelzpunkt (Reinigungsmittel) |
|---|---|---|---|---|---|
| $CH_3CH_2CH_2$- | | | | n-Propanol 100° | 144,5 - 146,5° (aus Ethanol) |
| $CH_3$- | $CH_3$- | $CH_3C_6H_5SO_3^{(-)}$ | 1 | Toluol 60° | 222 - 225° |
| $CH_3$- | $C_2H_5$- | $C_2H_5SO_3^{(-)}$ | 1 | Diethylsulfat 50° | 198 - 200° (aus Methanol) |
| $CH_3$- | $HOCH_2CH_2$- | $Cl^{(-)}$ | 1 | 2-Chlorethanol 128° | 218 - 220° u. Zers. (aus Ethanol) |
| $CH_3$- | $CH_3(CH_2)_3$- | $Br^{(-)}$ | 1 | n-Butylbromid 102° | 192 - 193° u. Zers. (aus Isopropanol) |
| $C_2H_5$- | $CH_3$- | $CH_3OSO_3^{(-)}$ | 1 | Toluol 60° | 233 - 237° u. Zers. (aus n-Butanol) |
| $CH_3OCH_2CH_2$- | $CH_3$- | $CH_3OSO_3^{(-)}$ | 1 | Toluol 60° | 220 - 225° |
| $C_2H_5$ | | | | Ethanol/NaOH 78° | 144 - 146° (aus Cyclohexan) |
| $CH_3OCH_2CH_2$- | | | | Methylglykol 80° | 116 - 118° (aus Cyclohexan) |

**Ansprüche** (für die Vertragsstaaten : BE, CH, DE, FR, GB, IT, LI, NL, SE)

1. Kationische Verbindungen der allgemeinen Formel (1)

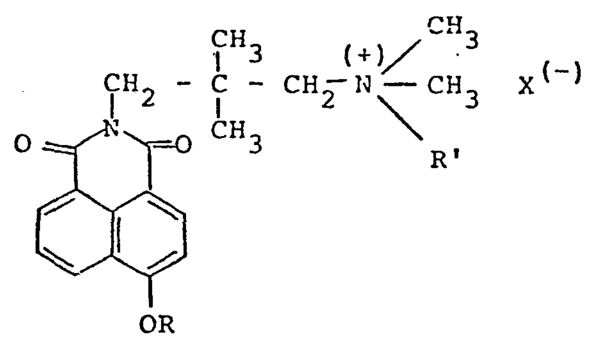

(1)

in welcher

R Alkyl, Hydroxyalkyl, Alkoxyalkyl, Phenylalkyl oder Phenoxyalkyl,

R' Alkyl, Hydroxyalkyl, Benzyl, Allyl und

$X^{(-)}$ ein farbloses Anion

bedeutet, wobei die Alkyl- und Alkoxygruppen jeweils 1 bis 4 C-Atome enthalten.

2. Verfahren zur Herstellung der Verbindungen der Formel (1) gemäss Anspruch 1, dadurch gekennzeichnet, daß man eine Verbindung der Formel (2)

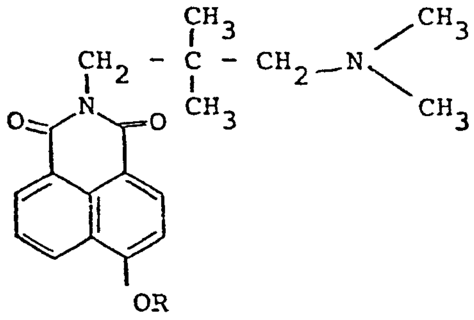

(2)

mit einer Verbindung der Formel

X—R′

alkyliert.

3. Verwendung der Verbindungen der Formel (1) gemäss Anspruch 1, als optische Aufheller, insbesondere für Polyacrylnitrilfasern.

**Ansprüche** (für den Vertragsstaat AT)

1. Verfahren zur Herstellung der Verbindungen der allgemeinen Formel (1)

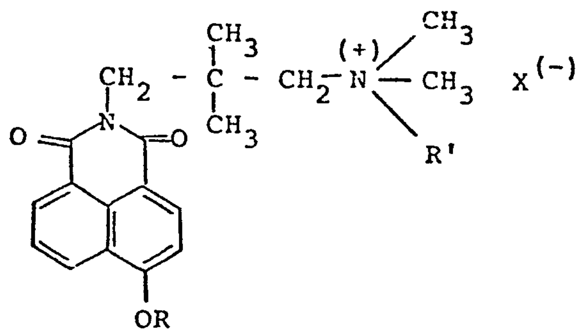

(1)

in welcher

R Alkyl, Hydroxyalkyl, Alkoxyalkyl, Phenylalkyl oder Phenoxyalkyl,
R′ Alkyl, Hydroxyalkyl, Benzyl, Allyl und
$X^{(-)}$ ein farbloses Anion

bedeutet, wobei die Alkyl- und Alkoxygruppen jeweils 1 bis 4 C-Atome enthalten, dadurch gekennzeichnet, daß man eine Verbindung der Formel (2)

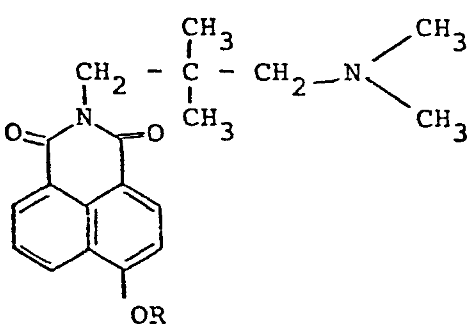

(2)

mit einer Verbindung der Formel

X—R′

alkyliert.

2. Verwendung der Verbindungen der Formel (1) gemäss Anspruch 1 als optische Aufheller, insbesondere für Polyacrylnitrilfasern.

**Claims** (for the Contracting States : BE, CH, DE, FR, GB, IT, LI, NL, SE)

1. Cationic compounds of the general formula 1

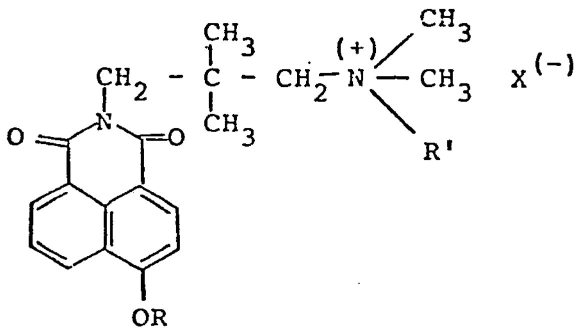

(1)

in which

R denotes alkyl, hydroxyalkyl, alkoxyalkyl, phenylalkyl, or phenoxyalkyl,

R′ denotes alkyl, hydroxyalkyl, benzyl, or allyl and

$X^{(-)}$ denotes a colorless anion, the alkyl and alkoxy groups each having 1 to 4 C-atoms.

2. Process for the manufacture of compounds of the formula (1) as claimed in claim 1, which comprises alkylating a compound of the formula (2)

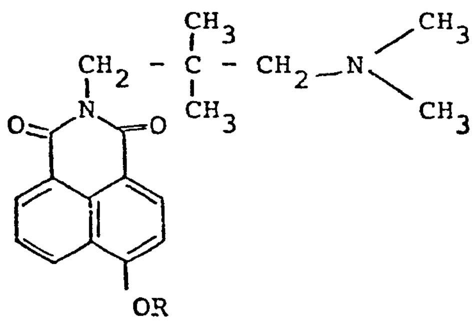

(2)

with a compound of the formula

X—R′.

3. Method of use of the compounds of the formula (1) as claimed in claim 1 as optical brighteners, especially for polyacrylonitrilefibers.

**Claims** (for the Contracting State AT)

1. Process for the manufacture of compounds of the general formula 1

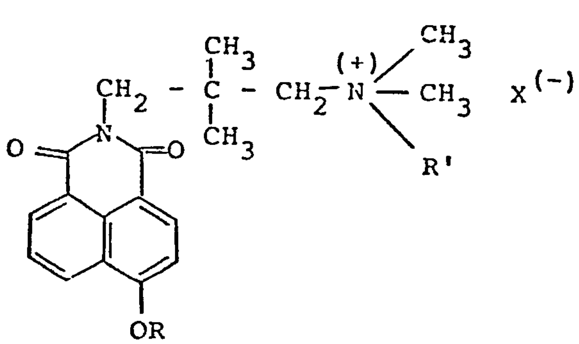

(1)

in which

R denotes alkyl, hydroxyalkyl, alkoxyalkyl, phenylalkyl, or phenoxyalkyl,

R′ denotes alkyl, hydroxyalkyl, benzyl, or allyl and

$X^{(-)}$ denotes a colorless anion, the alkyl and alkoxy groups each having 1 to 4 C-atoms, which comprises alkylating a compound of the formula (2)

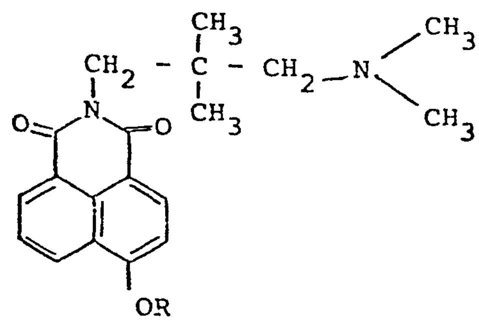

(2)

with a compound of the formula

X—R'.

2. Method of use of the compounds of the formula (1) according to claim 1 as optical brighteners, especially for polyacrylonitrilefibers.

**Revendications** (pour les Etats Contractants : BE, CH, DE, FR, GB, IT, LI, NL, SE)

1. Composés cationiques répondant à la formule générale (1)

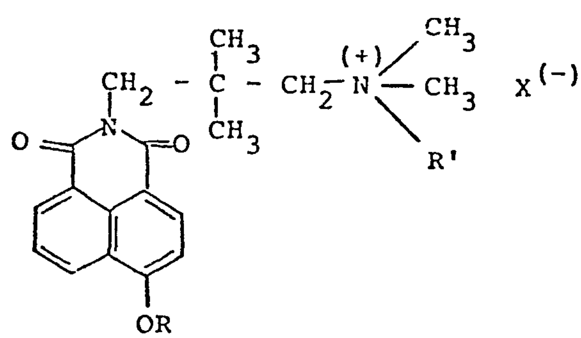

(1)

dans laquelle

R représente un radical alkyle, hydroxyalkyle, alcoxyalkyle, phénylalkyle ou phénoxyalkyle,

R' représente un radical alkyle, hydroxyalkyle, benzyle ou allyle et

$X^{(-)}$ représente un anion incolore,

les radicaux alkyles et alcoxy contenant chacun de 1 à 4 atomes de carbone.

2. Procédé de préparation des composés de formule (1) selon la revendication 1, procédé caractérisé en ce qu'on alkyle un composé de formule (2)

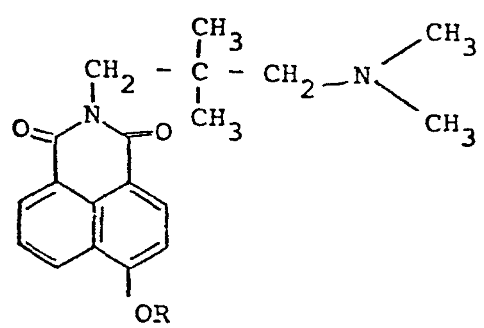

(2)

avec un composé de formule

X—R'.

3. Application des composés de formule (1) selon la revendication 1 comme azureurs optiques, plus particulièrement pour fibres en polyacrylonitrile.

**Revendications** (pour l'Etat contractant AT)

1. Procédé de préparation de composés répondant à la formule générale (1)

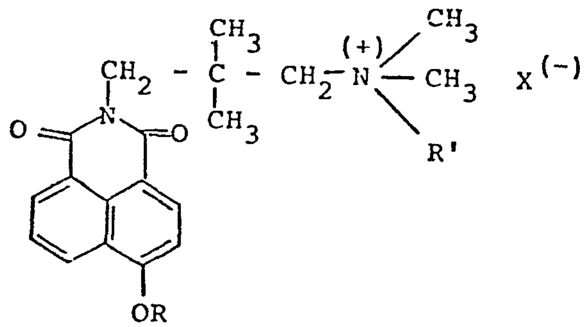

(1)

dans laquelle

R représente un radical alkyle, hydroxyalkyle, alcoxyalkyle, phénylalkyle ou phénoxyalkyle,

R' représente un radical alkyle, hydroxyalkyle, benzyle ou allyle et

$X^{(-)}$ représente un anion incolore,

les radicaux alkyles et alcoxy contenant chacun de 1 à 4 atomes de carbone, procédé caractérisé en ce qu'on alkyle un composé de formule (2)

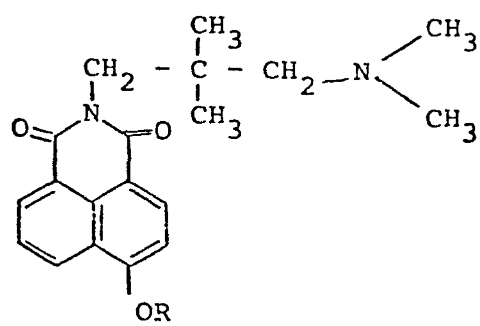

(2)

avec un composé de formule

X—R'.

2. Application des composés de formule (1) selon la revendication 1 comme azureurs optiques, plus particulièrement pour des fibres en polyacrylonitrile.